(19) **Europäisches Patentamt European Patent Office Office européen des brevets**

(11) **EP 4 579 685 B1**

(12) **EUROPEAN PATENT SPECIFICATION**

(45) Date of publication and mention of the grant of the patent:
**03.06.2026 Bulletin 2026/23**

(21) Application number: **23857568.2**

(22) Date of filing: **17.07.2023**

(51) International Patent Classification (IPC):
*G16H 50/70* (2018.01)   *G16H 30/20* (2018.01)
*G16H 30/40* (2018.01)   *G16H 50/20* (2018.01)
*G16H 50/30* (2018.01)   *G06V 10/70* (2022.01)
*A61B 1/233* (2006.01)   *A61B 1/00* (2006.01)
*A61B 6/03* (2006.01)   *A61B 6/00* (2024.01)
*G06T 7/00* (2017.01)   *G06V 20/69* (2022.01)
*G16H 40/63* (2018.01)

(52) Cooperative Patent Classification (CPC):
A61B 1/000094; A61B 1/000096; A61B 1/233;
A61B 6/00; A61B 6/032; A61B 6/501;
A61B 6/5217; A61B 6/5247; G06V 10/70;
G06V 20/698; G16H 30/20; G16H 30/40;
G16H 40/63; G16H 50/20; G16H 50/30;   (Cont.)

(86) International application number:
**PCT/KR2023/010178**

(87) International publication number:
**WO 2024/043531 (29.02.2024 Gazette 2024/09)**

(54) **TRAINING METHOD AND TRAINING APPARATUS OF MODEL FOR NASAL CAVITY MASS DETERMINATION, AND NASAL CAVITY MASS DETERMINATION METHOD AND APPARATUS**

TRAININGSVERFAHREN UND TRAININGSVORRICHTUNG EINES MODELLS ZUR BESTIMMUNG DER NASENHÖHLENMASSE SOWIE VERFAHREN UND VORRICHTUNG ZUR BESTIMMUNG DER NASENHÖHLENMASSE

PROCÉDÉ D'ENTRAÎNEMENT ET APPAREIL D'ENTRAÎNEMENT DE MODÈLE À DES FINS DE DÉTERMINATION DE MASSE DE CAVITÉ NASALE, ET PROCÉDÉ ET APPAREIL DE DÉTERMINATION DE MASSE DE CAVITÉ NASALE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **26.08.2022 KR 20220107724**

(43) Date of publication of application:
**02.07.2025 Bulletin 2025/27**

(73) Proprietor: **Korea University Research and Business Foundation Seoul 02841 (KR)**

(72) Inventors:
• **KIM, Tae-hoon Seoul 06520 (KR)**
• **CHO, Yongwon Seoul 05274 (KR)**
• **BYUN, Junhyoung Uijeongbu-si Gyeonggi-do 11750 (KR)**

(74) Representative: **Berggren Oy P.O. Box 16 Fabianinkatu 21 00101 Helsinki (FI)**

(56) References cited:
WO-A2-2021/054477   CN-A- 114 372 951
KR-A- 20190 103 937   KR-A- 20200 045 557
KR-A- 20200 121 558   KR-A- 20220 102 672

EP 4 579 685 B1

**(Cont. next page)**

- **GIRDLER BENTON ET AL: "Feasibility of a deep learning-based algorithm for automated detection and classification of nasal polyps and inverted papillomas on nasal endoscopic images", INTERNATIONAL FORUM OF ALLERGY & RHINOLOGY, vol. 11, no. 12, 20 June 2021 (2021-06-20), Oxford, pages 1637 - 1646, XP093325568, ISSN: 2042-6976, DOI: 10.1002/alr.22854**
- **OZCAN C ET AL: "Recurrent inverted papilloma of a pediatric patient: Clinico-radiological considerations", INTERNATIONAL JOURNAL OF PEDIATRIC OTORHINOLARYNGOLOGY, ELSEVIER, AMSTERDAM, NL, vol. 69, no. 6, 1 June 2005 (2005-06-01), pages 861 - 864, XP004881856, ISSN: 0165-5876, DOI: 10.1016/J.IJPORL.2005.01.033**
- **SEGAL NILI ET AL: "Nose biopsy: a comparison between two sampling techniques", EUROPEAN ARCHIVES OF OTO-RHINO-LARYNGOLOGY, SPRINGER BERLIN HEIDELBERG, BERLIN/HEIDELBERG, vol. 273, no. 6, 29 August 2015 (2015-08-29), pages 1445 - 1448, XP035679369, ISSN: 0937-4477, [retrieved on 20150829], DOI: 10.1007/S00405-015-3754-Y**
- **LÓPEZ FERNANDO ET AL: "The Impact of Histologic Phenotype in the Treatment of Sinonasal Cancer", ADVANCES IN THERAPY, HEALTH COMMUNICATIONS, METUCHEN, NJ, US, vol. 34, no. 10, 4 September 2017 (2017-09-04), pages 2181 - 2198, XP036348768, ISSN: 0741-238X, [retrieved on 20170904], DOI: 10.1007/S12325-017-0605-9**

(52) Cooperative Patent Classification (CPC): (Cont.)
**G16H 50/70;** G06T 7/0012; G06T 2207/10068; G06T 2207/10081; G06T 2207/20081; G06T 2207/20084; G06T 2207/30096; G06V 2201/031; G16H 40/67

## Description

## TECHNICAL FIELD

The following description relates to a model training technique for nasal cavity mass determination and a nasal cavity mass determination technique.

## BACKGROUND ART

When the appropriate treatment timing for an inverted papilloma is missed, destruction of surrounding tissues may occur and there is the potential for malignant transformation, making diagnosis crucial for timely and appropriate treatment. However, nasal polyps and inverted papillomas have similar appearances, which may cause difficulties in discriminating between nasal polyps and inverted papillomas during clinical examinations. Although techniques for detecting lesions within a nasal cavity exist, they have limitations such as low accuracy. Therefore, research is needed to overcome these limitations.

## DISCLOSURE OF THE INVENTION

## TECHNICAL SOLUTIONS

A training method of a model for determining a nasal cavity mass according to an embodiment includes extracting a plurality of patch images from a nasal endoscopy image comprising a nasal cavity region, training a neural network-based lesion detection model using the nasal endoscopy image and the plurality of patch images, detecting a presence of a lesion from the nasal endoscopy image using the trained lesion detection model, when it is detected that the lesion is present in the nasal endoscopy image, generating a combined image by combining the nasal endoscopy image with a computed tomography (CT) image of the nasal cavity region, and training, based on the generated combined image, a neural network-based determination model for determining nasal cavity masses related to the lesion.

The nasal endoscopy image may correspond to an entire image comprising the nasal cavity region, and the patch images may correspond to partial images of the entire image.

The extracting of the plurality of patch images may include extracting, from the nasal endoscopy image, the plurality of patch images corresponding to partial regions by randomly cropping the partial regions from an entire region of the nasal endoscopy image.

The training method according to an embodiment may further include, when a center of a lesion region is set in the nasal endoscopy image, extracting, from the nasal endoscopy image, the plurality of patch images corresponding to partial regions by cropping the partial regions based on a center of the lesion region.

The training method according to an embodiment may further include performing image processing on at least one of the extracted patch images and the nasal endoscopy image, the image processing comprising at least one of size enlargement, size reduction, rotation, and shifting, and wherein the training of the lesion detection model may include training the lesion detection model based on at least one of the extracted patch images and the nasal endoscopy image on which the image processing is performed.

The lesion detection model may be configured to classify whether the nasal endoscopy image is a lesion image comprising a lesion region or a normal image not comprising a lesion region.

The training of the lesion detection model may include assigning a weight to a texture and a shape of a nasal cavity region comprised in at least one of the nasal endoscopy image and the extracted plurality of patch images and performing, based on the texture and the shape of the nasal cavity region to which the weight is assigned, curriculum learning on the lesion detection model.

The determination model may be configured to classify the lesion as one of a nasal polyp, an inverted papilloma, or a normal tissue.

A nasal cavity mass determination method according to an embodiment includes extracting a plurality of patch images from a nasal endoscopy image comprising a nasal cavity region, detecting a presence of a lesion based on at least one of the nasal endoscopy image and the plurality of patch images using a lesion detection model, when it is detected that the lesion is present in the nasal endoscopy image, generating a combined image by combining the nasal endoscopy image with a CT image of the nasal cavity region, and performing, based on the generated combined image, nasal cavity mass determination for the lesion using a neural network-based determination model.

The nasal endoscopy image may correspond to an entire image comprising the nasal cavity region, and the patch images may correspond to partial images of the entire image.

The extracting of the plurality of patch images may include extracting, from the nasal endoscopy image, the plurality of patch images corresponding to partial regions by randomly cropping the partial regions from an entire region of the nasal endoscopy image.

The lesion detection model may be trained using the nasal endoscopy image and the plurality of patch images, and after being trained, configured to classify whether the nasal endoscopy image is a lesion image comprising a lesion region or a normal image not comprising a lesion region.

The lesion detection model may be configured to assign a weight to a texture and a shape of a nasal cavity region comprised in at least one of the nasal endoscopy image and the plurality of patch images and is trained based on the texture and the shape of the nasal cavity region to which the weight is assigned.

The lesion determination model may be trained based on the combined image generated by combining the nasal endoscopy image with a CT image of the nasal cavity region, and after being trained, configured to classify the lesion as one of a nasal polyp, an inverted papilloma, or a normal tissue.

A training apparatus of a model for determining a nasal cavity mass may include an image preprocessing part configured to extract a plurality of patch images from a nasal endoscopy image comprising a nasal cavity region, a lesion detection model training part configured to train a neural network-based lesion detection model using the nasal endoscopy image and the extracted plurality of patch images, a detector configured to detect a presence of a lesion from the nasal endoscopy image using the trained lesion detection model, a combined image generator configured to, when it is detected that the lesion is present in the nasal endoscopy image, generate a combined image by combining the nasal endoscopy image with a computerized tomography (CT) image of the nasal cavity region, and a determination model training part configured to train, based on the generated combined image, a neural network-based determination model for determining nasal cavity masses of the lesion.

The image preprocessing part may be configured to extract, from the nasal endoscopy image, the plurality of patch images corresponding to partial regions by randomly cropping the partial region from an entire region of the nasal endoscopy image.

The image preprocessing part may be configured to, when a center of a lesion region is set in the nasal endoscopy image, extract, from the nasal endoscopy image, the plurality of patch images corresponding to partial regions by cropping the partial regions based on the center of the lesion region.

The lesion detection model training part may be configured to assign a weight to a texture and a shape of a nasal cavity region comprised in at least one of the nasal endoscopy image and the extracted plurality of patch images and perform, based on the texture and the shape of the nasal cavity region to which the weight is assigned, curriculum learning on the lesion detection model.

A nasal cavity mass determination apparatus according to an embodiment includes an image preprocessing part configured to extract a plurality of patch images from a nasal endoscopy image comprising a nasal cavity region, a detector configured to detect a presence of a lesion based on at least one of the nasal endoscopy image and the plurality of patch images using a lesion detection model, a combined image generator configured to, when it is detected that the lesion is present in the nasal endoscopy image, generate a combined image by combining the nasal endoscopy image with a CT image of the nasal cavity region, and a nasal cavity mass determination part configured to perform nasal cavity mass determination on the lesion based on the generated combined image using a neural network-based determination model.

The lesion detection model may be configured to assign a weight to a texture and a shape of a nasal cavity region comprised in at least one of the nasal endoscopy image and the plurality of patch images and is trained through curriculum learning based on the texture and the shape of the nasal cavity region to which the weight is assigned.

EFFECTS OF THE INVENTION

According to an embodiment, the accuracy of nasal cavity mass determination may be improved by first detecting the presence of a lesion based on a nasal endoscopy image and then performing nasal cavity mass determination on a lesion in a second step based on both a computerized tomography (CT) image and the nasal endoscopy image.

According to an embodiment, nasal cavity masses that are generally difficult to determine may be automatically determined using a neural network-based model, thereby improving convenience and enhancing the accuracy of determination.

According to an embodiment, a supportive role in diagnosing a nasal cavity mass may be performed, thereby assisting a clinician in diagnosing a nasal endoscopy image and preventing unnecessary treatments by supporting accurate diagnosis.

**BRIEF DESCRIPTION OF DRAWINGS**

FIG. 1 is a diagram schematically illustrating a nasal cavity mass determination system according to an embodiment.
FIG. 2 is a flowchart illustrating a model training method for nasal cavity mass determination, according to an embodiment.
FIG. 3 is a flowchart illustrating a nasal cavity mass determination method according to an embodiment.
FIG. 4 is a diagram illustrating the process of extracting a plurality of patch images from a nasal endoscopy image, according to an embodiment.
FIG. 5 is a diagram illustrating a lesion detection model according to another embodiment.
FIG. 6 is a diagram illustrating the structure of a training apparatus according to an embodiment.
FIG. 7 is a diagram illustrating the structure of a nasal cavity mass determination apparatus, according to an embodiment.

**BEST MODE FOR CARRYING OUT THE INVENTION**

The following detailed structural or functional descriptions are provided as examples only, and various alterations and modifications may be made to the embodiments. Accordingly, the embodiments are not to be construed as limited to the disclosure and should be understood to include all changes, equivalents, or replacements within the idea and the technical scope of the disclosure.

Terms, such as first, second, and the like, may be used herein to describe components. Each of these terminologies is not used to define an essence, order or sequence of a corresponding component but used merely to distinguish the corresponding component from other component(s). For example, a first component may be referred to as a second component, and similarly the second component may also be referred to as the first component.

It should be noted that if one component is described as being "connected", "coupled", or "joined" to another component, a third component may be "connected", "coupled", and "joined" between the first and second components, although the first component may be directly connected, coupled, or joined to the second component.

The singular forms "a", "an", and "the" are intended to include the plural forms as well, unless the context clearly indicates otherwise. It should be understood that the terms "comprises/comprising" or "includes/including" when used herein, specify the presence of stated features, integers, steps, operations, elements, components, or groups thereof, but do not preclude the presence or addition of one or more other features, integers, steps, operations, elements, components, or groups thereof.

Unless otherwise defined, all terms used herein including technical or scientific terms have the same meaning as commonly understood by one of ordinary skill in the art to which this disclosure pertains. Terms, such as those defined in commonly used dictionaries, should be construed to have meanings matching with contextual meanings in the relevant art, and are not to be construed to have an ideal or excessively formal meaning unless otherwise defined herein.

Hereinafter, embodiments are described in detail with reference to the accompanying drawings. When describing the embodiments with reference to the accompanying drawings, like reference numerals refer to like elements and any repeated description related thereto will be omitted.

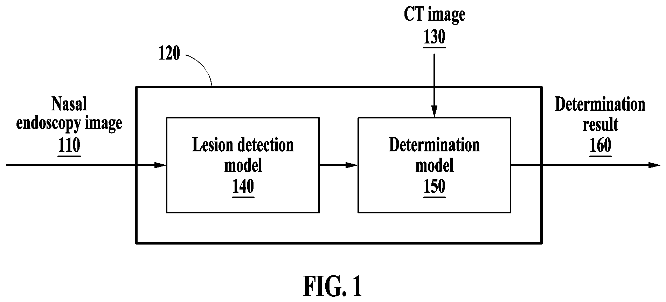

FIG. 1 is a diagram schematically illustrating a nasal cavity mass determination system according to an embodiment.

A nasal cavity mass determination system described herein may detect the presence of a lesion based on a nasal endoscopy image and, when it is detected that a lesion is present, determine the type of lesion based on both the nasal endoscopy image and a computed tomography (CT) image. The nasal cavity mass determination system described herein may improve the accuracy of nasal cavity mass determination by utilizing both the nasal endoscopy image and the CT image for determining a nasal cavity mass.

Referring to FIG. 1, a nasal cavity mass determination system 120 may include a lesion detection model 140 and a determination model 150. The lesion detection model 140 and the determination model 150 may be deep learning-based neural network models and may be trained using at least one method among iterative learning, transfer learning, and supervised learning.

The lesion detection model 140 may receive a nasal endoscopy image 110. Here, the nasal endoscopy image 110 may be an image on which image preprocessing is performed. Based on the nasal endoscopy image 110, the lesion detection model 140 may detect the presence of a lesion. When the lesion detection model 140 detects the presence of a lesion based on the nasal endoscopy image 110, the determination model 150 may receive both the nasal endoscopy image and a CT image of a nasal cavity region. Here, the nasal endoscopy image and the CT image of the nasal cavity region may be combined into a combined image. The determination model 150 may output a determination result 160 based on the nasal endoscopy image and the CT image of the nasal cavity region. The determination result 160 may include a result by classifying the lesion as one of a nasal polyp, an inverted papilloma, or a normal tissue. Additionally, the determination result 160 may also include respective probability values for whether the lesion corresponds to a nasal polyp, an inverted papilloma, or a normal tissue.

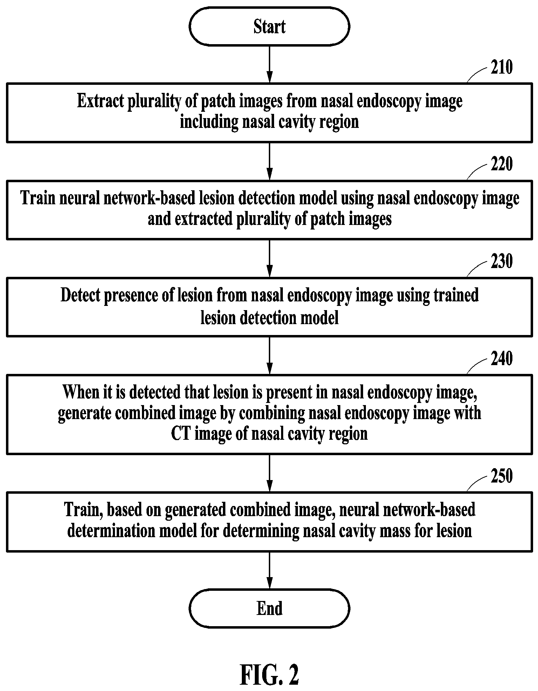

FIG. 2 is a flowchart illustrating a model training method for nasal cavity mass determination, according to an embodiment.

Referring to FIG. 2, a training apparatus may extract a plurality of patch images from a nasal endoscopy image including a nasal cavity region in operation 210. Here, the nasal endoscopy image may correspond to an entire image including the nasal cavity region and, as training data, may be classified into a training set, a tuning set, and a test set. The ratio for classifying the nasal endoscopy image into a training set, a tuning set, and a test set may, for example, be 7 for the training set, 1 for the tuning set, and 2 for the test set, and the classification may be performed randomly. A nasal endoscopy image classified into the training set may be used to train at least one of a lesion detection model and a determination model, which are described below. A nasal endoscopy image classified into the tuning set may be used to tune parameters to improve the performance of at least one of a lesion detection model and a determination model trained based on the nasal endoscopy image classified into the training set. Additionally, a nasal endoscopy image classified into the test set may be used to test the performance of at least one of the lesion detection model and the determination model. The classification of nasal endoscopy images is described as an example herein, but embodiments are not limited thereto.

Patch images may correspond to partial images of an entire image or partial images of a nasal endoscopy image. The process by which the training apparatus extracts a plurality of patch images from the nasal endoscopy image may also be referred to as an image preprocessing process. **In** an embodiment, the training apparatus may extract, from the nasal endoscopy image, the plurality of patch images corresponding to partial regions by randomly cropping the partial regions from the entire region of the nasal endoscopy image.

Additionally, in another embodiment, when the center of a lesion region is set in the nasal endoscopy image, the training apparatus may extract, from the nasal endoscopy image, a plurality of patch images corresponding to partial regions by cropping the partial regions based on the center of the lesion region. For example, when the center of the lesion region is set in the nasal endoscopy image based on a user input or a user selection provided by a health professional, the training apparatus may extract, from the nasal endoscopy image, a plurality of patch images corresponding to partial regions by cropping the partial regions including the center set as the center of the lesion region.

**In** an embodiment, the training apparatus may perform image preprocessing by performing image processing including at least one of size enlargement, size reduction, rotation, and shifting, on at least one of the extracted patch images and the nasal endoscopy image.

**In** operation 220, the training apparatus may train a neural network-based lesion detection model using the nasal endoscopy image and the extracted plurality of patch images. The training apparatus may train the lesion detection model based on at least one of the extracted patch images, on which image processing is performed, and the nasal endoscopy image. The training apparatus may assign a weight to the texture and shape of a nasal cavity region included in at least one of the nasal endoscopy image and the extracted plurality of patch images. For example, the training apparatus may be designed so that the lesion detection model learns the texture and shape of the nasal endoscopy image to detect the presence of a lesion by combining local information of the nasal endoscopy image with global information of the nasal endoscopy image. Here, the local information may include predetermined information obtained from local information, while the global information may include the overall shape and texture of a lesion.

The training apparatus may assign a weight to the texture and shape of the nasal cavity region determined to have a predetermined characteristic based on predetermined criteria. Based on the texture and shape of the nasal cavity region to which the weight is assigned, the training apparatus may perform curriculum learning on the lesion detection model. By assigning a weight to the texture and shape of the nasal cavity region, the training apparatus may reflect various lesion textures and shapes during the process of training the lesion detection model.

In another embodiment, the training apparatus may train the lesion detection model based on patch images extracted by cropping partial regions based on the center of a lesion region. In this case, lesion-centered curriculum learning may be performed. Lesion-centered curriculum learning may be useful for training a deep learning model and interpreting the location and characteristic of a lesion based on limited medical datasets (a nasal endoscopy image or other data).

In operation 230, the training apparatus may detect the presence of a lesion from the nasal endoscopy image using the trained lesion detection model. In an embodiment, the training apparatus may classify whether the nasal endoscopy image is a lesion image including a lesion region or a normal image not including a lesion region. When it is determined that the nasal endoscopy image is the lesion image including a lesion region, the training apparatus may detect the presence of a lesion in the nasal endoscopy image.

When it is detected that a lesion is present in the nasal endoscopy image, the training apparatus may obtain the region including the lesion from the nasal endoscopy image and perform segmentation. In operation 240, the training apparatus may generate a combined image by combining the nasal endoscopy image with a CT image of the nasal cavity region.

In operation 250, the training apparatus may train, based on the generated combined image, a neural network-based determination model for determining nasal cavity masses. The determination model may classify a lesion as one of a nasal polyp, an inverted papilloma, and a normal tissue. In an embodiment, the training apparatus may train the determination model using the combined image based on at least one of transfer learning and curriculum learning.

The training apparatus described herein may train a nasal cavity mass determination model that may more accurately determine nasal cavity masses by utilizing both a three-dimensional (3D) CT image and a 2D nasal endoscopy image.

The attention mechanism of at least one of the trained lesion detection model and the determination model may particularly align with a local lesion-related region that an experienced otolaryngologist focuses on during a nasal endoscopy examination.

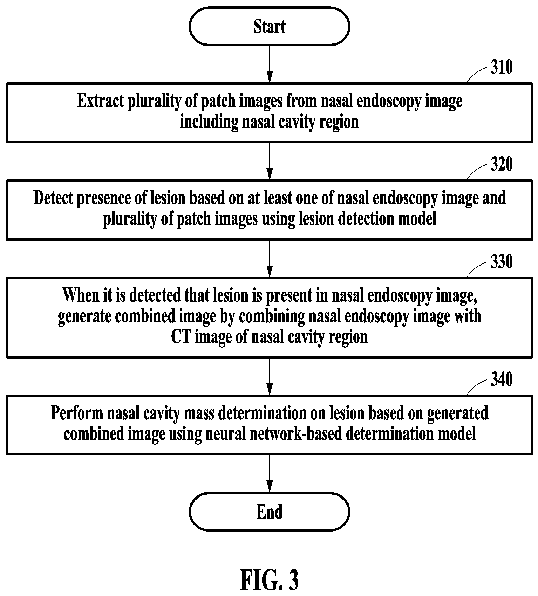

FIG. 3 is a flowchart illustrating a nasal cavity mass determination method according to an embodiment.

Referring to FIG. 3, a nasal cavity mass determination apparatus may extract a plurality of patch images from a nasal endoscopy image including a nasal cavity region. Operation 310 may be similar to operation 210 described in detail with reference to FIG. 2. The nasal endoscopy image may correspond to the entire image including the nasal cavity region, and the patch images may correspond to partial images of the entire image.

The nasal cavity mass determination apparatus may extract, from the nasal endoscopy image, a plurality of patch images corresponding to partial regions by randomly cropping the partial regions from the entire region of the nasal endoscopy image. Additionally, in another embodiment, when the center of a lesion region is set in the nasal endoscopy image based on a user input or a user selection provided by a health professional, the nasal cavity mass determination apparatus may also extract, from the nasal endoscopy image, a plurality of patch images corresponding to partial regions by cropping the partial regions including the center set as the center of the lesion region.

In operation 320, the nasal cavity mass determination apparatus may detect the presence of a lesion based on at least one of the nasal endoscopy image and the plurality of patch images using a lesion detection model. In an embodiment, the lesion detection model may be trained using the nasal endoscopy image and the plurality of patch images. The lesion detection model may assign a weight to the texture and shape of the nasal cavity region included in at least one of the nasal endoscopy image and the plurality of patch images and may be trained through curriculum learning based on the texture and shape of the nasal cavity region to which the weight is assigned. After being trained, the lesion detection model may classify whether the nasal endoscopy image is a lesion image including a lesion region or a normal image not including a lesion region. Operation 320 may be similar to operation 230 described in detail with reference to FIG. 2.

In operation 330, when it is detected that a lesion is present in the nasal endoscopy image, the nasal cavity mass determination apparatus may generate a combined image by combining the nasal endoscopy image with the CT image of the nasal cavity region. Operation 330 may be similar to operation 240 described in detail with reference to FIG. 2.

In operation 250, the nasal cavity mass determination apparatus may perform nasal cavity mass determination on a lesion based on the generated combined image using a neural network-based determination model.

The determination model may be trained, based on the combined image generated by combining the nasal endoscopy image with the CT image of the nasal cavity region included in training data. After being trained, the determination model may perform nasal cavity mass determination on a lesion by classifying the lesion as one of a nasal polyp, an inverted papilloma, or a normal tissue.

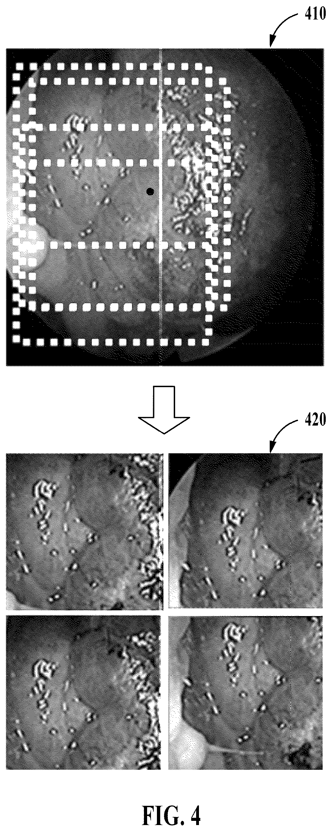

FIG. 4 is a diagram illustrating the process of extracting a plurality of patch images from a nasal endoscopy image, according to an embodiment.

Both the training apparatus and the nasal cavity mass determination apparatus described herein may extract a plurality of patch images 420 from a nasal endoscopy image 410. The nasal endoscopy image 410 may correspond to an entire image including a nasal cavity region, and the patch images 420 may correspond to partial images of the entire image or partial images of the nasal endoscopy image 410.

The training apparatus or the nasal cavity mass determination apparatus may extract, from the nasal endoscopy image 410, the plurality of patch images 420 corresponding to partial regions by randomly cropping the partial regions from the entire region of the nasal endoscopy image 410. Additionally, in another embodiment, when the center of a lesion region is set in the nasal endoscopy image 410 based on a user input or a user selection provided by a health professional, the nasal cavity mass determination apparatus or the training apparatus may also extract, from the nasal endoscopy image 410, the plurality of patch images 420 corresponding to partial regions by cropping the partial regions including the center set as the center of the lesion region.

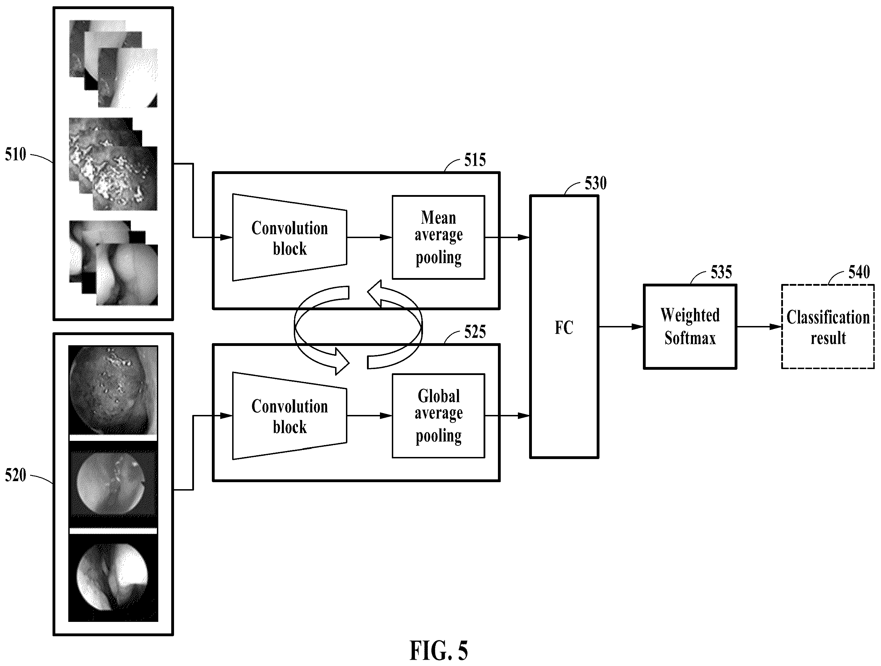

FIG. 5 is a diagram illustrating a lesion detection model according to another embodiment.

Referring to FIG. 5, a lesion detection model may include a first lesion detection model 515 that receives a patch image 510 extracted from a nasal endoscopy image and a second lesion detection model 525 that receives a nasal endoscopy image 520. Here, the patch image 510 may have a resolution of, for example, 256*256 and the nasal endoscopy image 520 may have a resolution of, for example, 512*512. The first lesion detection model 515 may include a convolutional model and average pooling (mean pooling), while the second lesion detection model 525 may include a convolutional model and average pooling (Global average pooling).

At least one of the first lesion detection model 515 and the second lesion detection model 525 may include InceptionResNetV2 to detect, from a nasal endoscopy image, a lesion including at least one of a nasal polyp, an inverted papilloma, or a normal tissue. InceptionResNetV2 may include residual connections including various convolutional blocks (or convolutional filters) connected to an initial layer and a residual block. InceptionResNetV2 may prevent a gradient descent problem that may occur due to the depth of a deep learning network and also reduce training time.

The first lesion detection model 515 and the second lesion detection model 525 may be trained through curriculum learning while sharing a weight or through transfer learning. The InceptionResNetV2 of the first lesion detection model 515 and the second lesion detection model 525 may first be trained based on ImageNet Large Scale Visual Recognition Competition 25 (ILSVRC 25). Such a convolution-based neural network is not trained from scratch but may learn general or other medical image functions based on datasets from other domains. Subsequently, the first lesion detection model 515 and the second lesion detection model 525 may be trained using all the images included in training data by fine-tuning only the last layer in a pre-trained state.

According to a prior art, a full-resolution nasal endoscopy image includes a complex pattern, including lesions, organs, and tissues, which may make training with limited datasets challenging. The lesion detection model described herein may receive the patch images 510 extracted from the nasal endoscopy image 520 to improve the training and adjustment of the prior art. The patch images 510 may correspond to half the original resolution of the nasal endoscopy image 520. To compensate for the discrepancies between the patch images 510 and the nasal endoscopy image 520, the lesion detection model may perform fine-tuning on parameters included in a network of the lesion detection model using the nasal endoscopy image 520 with a full resolution.

The trained first lesion detection model 515 may receive the patch images 510 and output an output value by inputting the received patch images 510 to a convolutional model and average pooling. In addition, the trained second lesion detection model 525 may receive the nasal endoscopy image 520 and obtain an output value by inputting the received nasal endoscopy image 520 to the convolutional model and average pooling.

The output value output from the first lesion detection model 515 and the output value output from the second lesion detection model 525 may be produced as a classification result 540 by being processed through a fully connected (FC) layer 530 and a weighted Softmax function 535. The classification result 540 may include, for example, a result of classifying a lesion as one of a nasal polyp, an inverted papilloma, or a normal tissue and include a probability value for the lesion being one of a nasal polyp, an inverted papilloma, or a normal tissue. A binary classification cross-entropy cost function related to the probability value may be expressed by the equation below.

[Equation 1]

$$\text{Loss}(y, f) = -y \log f - (1 - y) \log (1 - f)$$

Here, f and y may denote an inferred probability value and a desired output corresponding to an output value, respectively.

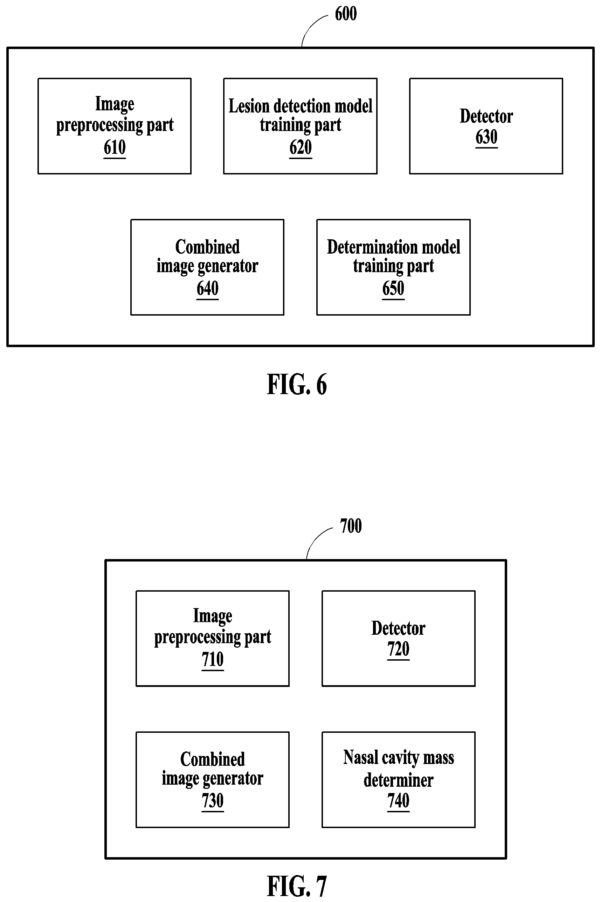

FIG. 6 is a diagram illustrating the structure of a training apparatus according to an embodiment.

Referring to FIG. 6, a training apparatus 600 may include an image preprocessing part 610, a lesion detection model training part 620, a detector 630, a combined image generator 640, and a determination model training part 650. Here, the training apparatus 600 may correspond to the training apparatus described herein.

The image preprocessing part 610 may extract a plurality of patch images from a nasal endoscopy image including a nasal cavity region. The image preprocessing part 610 may extract, from the nasal endoscopy image, the plurality of patch images corresponding to partial regions by randomly cropping the partial regions from the entire region of the nasal endoscopy image. In another embodiment, when the center of a lesion region is set in the nasal endoscopy image, the image preprocessing part 610 may extract, from the nasal endoscopy image, the plurality of patch images corresponding to partial regions by cropping the partial regions based on the center of the lesion region.

The lesion detection model training part 620 may train a neural network-based lesion detection model using the nasal endoscopy image and the extracted plurality of patch images. The lesion detection model training part 620 may assign a weight to the texture and shape of the nasal cavity region included in at least one of the nasal endoscopy image and the extracted plurality of patch images and train the lesion detection model through curriculum learning based on the texture and shape of the nasal cavity region to which the weight is assigned.

The detector 630 may detect the presence of a lesion from the nasal endoscopy image using the trained lesion detection model.

The combined image generator 640 may generate a combined image by combining the nasal endoscopy image with a CT image of the nasal cavity region when it is detected that a lesion is present in the nasal endoscopy image.

The discrimination model training part 650 may train a neural network-based determination model for determining nasal masses related to a lesion, based on the generated combined image.

The operations performed by the image preprocessing part 610, the lesion detection model training part 620, the detector 630, the combined image generator 640, and the determination model training part 650 described above may be performed by an apparatus including one or more processors and a memory. For example, the operations of the preprocessing part 610, the lesion detection model training part 620, the detector 630, the combined image generator 640, and the determination model training part 650 may be performed by a processor executing instructions stored in a memory.

FIG. 7 is a diagram illustrating the structure of a nasal cavity mass determination apparatus, according to an embodiment.

Referring to FIG. 7, a nasal cavity mass determination apparatus 700 may include an image preprocessing part 710, a detector 720, a combined image generator 730, and a nasal cavity mass determiner 740. Here, the nasal cavity mass determination apparatus 700 may correspond to the nasal cavity mass determination apparatus described herein.

The image preprocessing part 710 may extract, from a nasal endoscopy image, a plurality of patch images including a nasal cavity region. Additionally, the detector 720 may detect the presence of a lesion based on at least one of the nasal endoscopy image and the plurality of patch images using a lesion detection model.

The combined image generator 730 may generate a combined image by combining the nasal endoscopy image with a CT image of the nasal cavity region when it is detected that a lesion is present in the nasal endoscopy image, and the nasal mass determiner 740 may perform nasal cavity mass determination on the lesion based on the generated combined image using a neural network-based determination model.

The operations performed by the image preprocessing part 710, the detector 720, the combined image generator 730, and the nasal cavity mass determiner 740 described above may be performed by an apparatus including one or more processors and a memory. For example, the operations of the image preprocessing part 710, the detector 720, the combined image generator 730, and the nasal cavity mass determiner 740 may be performed by a processor executing instructions stored in the memory.

The embodiments described herein may be implemented using a hardware component, a software component, and/or a combination thereof. A processing device may be implemented using one or more general-purpose or special-purpose computers, such as, for example, a processor, a controller and an arithmetic logic unit (ALU), a digital signal processor (DSP), a microcomputer, a field programmable gate array (FPGA), a programmable logic unit (PLU), a microprocessor or any other device capable of responding to and executing instructions in a defined manner. The processing device may run an operating system (OS) and one or more software applications that run on the OS. The processing device may also access, store, manipulate, process, and generate data in response to execution of the software. For purpose of simplicity, the description of the processing device is used as singular; however, one skilled in the art will appreciate that the processing device may include a plurality of processing elements and/or multiple types of processing elements. For example, the processing device may include a plurality of processors or a single processor and a single controller. In addition, different processing configurations are possible, such as parallel processors.

The software may include a computer program, a piece of code, an instruction, or one or more combinations thereof, to independently or collectively instruct or configure the processing device to operate as desired. Software and data may be embodied permanently or temporarily in any type of machine, component, physical or pseudo equipment, computer storage medium or device, or in a propagated signal wave capable of providing instructions or data to or being interpreted by the processing device. The software may also be distributed over network-coupled computer systems so that the software is stored and executed in a distributed fashion. The software and data may be stored in a non-transitory computer-readable recording medium.

The methods according to the above-described embodiments may be recorded in non-transitory computer-readable media including program instructions to implement various operations of the above-described embodiments. The media may also include, alone or in combination with the program instructions, data files, data structures, and the like. The program instructions recorded on the media may be those specially designed and constructed for the purposes of examples, or they may be of the kind well-known and available to those having skill in the computer software arts. Examples of non-transitory computer-readable media include magnetic media such as hard disks, floppy disks, and magnetic tape; optical media such as CD-ROM discs or DVDs; magneto-optical media such as floptical disks; and hardware devices that are specially configured to store and perform program instructions, such as read-only memory (ROM), random access memory (RAM), flash memory, and the like. Examples of program instructions include both machine code, such as produced by a compiler, and files containing higher-level code that may be executed by the computer using an interpreter.

The above-described hardware devices may be configured to act as one or more software modules in order to perform the operations of the above-described embodiments, or vice versa.

As described above, although the examples have been described with reference to the limited drawings, a person skilled in the art may apply various technical modifications and variations based thereon. For example, suitable results may be achieved if the described techniques are performed in a different order, and/or if components in a described system, structure, device, or circuit are combined in a different manner, and/or replaced or supplemented by other components or their equivalents.

Therefore, other implementations and other embodiments are also within the scope of the following claims.

## Claims

1.  A training method of a model for determining a nasal cavity mass, the training method comprising:

    extracting a plurality of patch images from a nasal endoscopy image comprising a nasal cavity region;
    training a neural network-based lesion detection model using the nasal endoscopy image and the plurality of patch images;
    detecting a presence of a lesion from the nasal endoscopy image using the trained lesion detection model;
    when it is detected that the lesion is present in the nasal endoscopy image, generating a combined image by combining the nasal endoscopy image with a computed tomography image of the nasal cavity region; and training, based on the generated combined image, a neural network-based determination model for determining nasal cavity masses related to the lesion.

2.  The training method of claim 1, wherein

    the nasal endoscopy image corresponds to an entire image comprising the nasal cavity region, and

the patch images correspond to partial images of the entire image.

3. The training method of claim 1, wherein the extracting of the plurality of patch images comprises extracting, from the nasal endoscopy image, the plurality of patch images corresponding to partial regions by randomly cropping the partial regions from an entire region of the nasal endoscopy image.

4. The training method of claim 1, further comprising: when a center of a lesion region is set in the nasal endoscopy image, extracting, from the nasal endoscopy image, the plurality of patch images corresponding to partial regions by cropping the partial regions based on a center of the lesion region.

5. The training method of claim 1, further comprising:

performing image processing on at least one of the extracted patch images and the nasal endoscopy image, the image processing comprising at least one of size enlargement, size reduction, rotation, and shifting, and

wherein the training of the lesion detection model comprises training the lesion detection model based on at least one of the extracted patch images and the nasal endoscopy image on which the image processing is performed.

6. The training method of claim 1, wherein the lesion detection model is configured to classify whether the nasal endoscopy image is a lesion image comprising a lesion region or a normal image not comprising a lesion region, and

wherein the determination model is configured to classify the lesion as one of a nasal polyp, an inverted papilloma, or a normal tissue.

7. The training method of claim 1, wherein the training of the lesion detection model comprises:

assigning a weight to a texture and a shape of a nasal cavity region comprised in at least one of the nasal endoscopy image and the extracted plurality of patch images; and

performing, based on the texture and the shape of the nasal cavity region to which the weight is assigned, curriculum learning on the lesion detection model.

8. A nasal cavity mass determination method comprising:

extracting a plurality of patch images from a nasal endoscopy image comprising a nasal cavity region;

detecting a presence of a lesion based on at least one of the nasal endoscopy image and the plurality of patch images using a lesion detection model;

when it is detected that the lesion is present in the nasal endoscopy image, generating a combined image by combining the nasal endoscopy image with a computed tomography, CT, image of the nasal cavity region; and

performing, based on the generated combined image, nasal cavity mass determination for the lesion using a neural network-based determination model.

9. The nasal cavity mass determination method of claim 8, wherein

the nasal endoscopy image corresponds to an entire image comprising the nasal cavity region, and

the patch images correspond to partial images of the entire image.

10. The nasal cavity mass determination method of claim 8, wherein the extracting of the plurality of patch images comprises extracting, from the nasal endoscopy image, the plurality of patch images corresponding to partial regions by randomly cropping the partial regions from an entire region of the nasal endoscopy image.

11. The nasal cavity mass determination method of claim 8, wherein the lesion detection model is

trained using the nasal endoscopy image and the plurality of patch images, and

after being trained, configured to classify whether the nasal endoscopy image is a lesion image comprising a lesion region or a normal image not comprising a lesion region.

12. The nasal cavity mass determination method of claim 8, wherein the lesion detection model is configured to assign a weight to a texture and a shape of a nasal cavity region comprised in at least one of the nasal endoscopy image and the plurality of patch images and is trained based on the texture and the shape of the nasal cavity region to which the weight is assigned.

13. The nasal cavity mass determination method of claim 8, wherein the neural network-based determination model is

trained based on the combined image generated by combining the nasal endoscopy image with a CT image of the nasal cavity region, and

after being trained, configured to classify the lesion as one of a nasal polyp, an inverted papilloma, or a normal tissue.

14. A training apparatus of a model for determining a nasal cavity mass, the training apparatus comprising:

an image preprocessing part configured to extract a plurality of patch images from a nasal endoscopy image comprising a nasal cavity region;
a lesion detection model training part configured to train a neural network-based lesion detection model using the nasal endoscopy image and the extracted plurality of patch images;
a detector configured to detect a presence of a lesion from the nasal endoscopy image using the trained lesion detection model;
a combined image generator configured to, when it is detected that the lesion is present in the nasal endoscopy image, generate a combined image by combining the nasal endoscopy image with a computerized tomography image of the nasal cavity region; and
a determination model training part configured to train, based on the generated combined image, a neural network-based determination model for determining nasal cavity masses of the lesion.

15. A nasal cavity mass determination apparatus comprising:

an image preprocessing part configured to extract a plurality of patch images from a nasal endoscopy image comprising a nasal cavity region;
a detector configured to detect a presence of a lesion based on at least one of the nasal endoscopy image and the plurality of patch images using a lesion detection model;
a combined image generator configured to, when it is detected that the lesion is present in the nasal endoscopy image, generate a combined image by combining the nasal endoscopy image with a computerized tomography image of the nasal cavity region; and
a nasal cavity mass determination part configured to perform nasal cavity mass determination on the lesion based on the generated combined image using a neural network-based determination model.

**Patentansprüche**

1. Trainingsverfahren eines Modells zum Bestimmen einer Nasenhöhlenmasse, das Trainingsverfahren umfassend:

Extrahieren einer Vielzahl von Pflasterbildern aus einem nasalen Endoskopiebild, umfassend eine Nasenhöhlenregion;
Training eines auf einem neuronalen Netzwerk basierenden Läsionserkennungsmodells unter Verwendung des nasalen Endoskopiebildes und der Vielzahl von Pflasterbildern;
Erkennen des Vorhandenseins einer Läsion aus dem nasalen Endoskopiebild unter Verwendung des trainierten Läsionserkennungsmodells;
wenn erkannt wird, dass die Läsion in dem nasalen Endoskopiebild vorhanden ist, Erzeugen eines kombinierten Bildes durch Kombinieren des nasalen Endoskopiebildes mit einem Computertomographiebild der Nasenhöhlenregion; und
Trainieren, basierend auf dem erzeugten kombinierten Bild, eines auf einem neuronalen Netzwerk basierenden Bestimmungsmodells zum Bestimmen von mit der Läsion verbundenen Nasenhöhlenmassen.

2. Trainingsverfahren nach Anspruch 1, wobei

das nasale Endoskopiebild einem gesamten Bild entspricht, das die Nasenhöhlenregion umfasst, und
die Pflasterbilder Teilbildern des gesamten Bildes entsprechen.

3. Trainingsverfahren nach Anspruch 1, wobei das Extrahieren der Vielzahl von Pflasterbildern das Extrahieren der Vielzahl von Pflasterbildern, die Teilregionen entsprechen, aus dem nasalen Endoskopiebild umfasst, indem die Teilregionen zufällig aus einer gesamten Region des nasalen Endoskopiebildes ausgeschnitten werden.

4. Trainingsverfahren nach Anspruch 1, ferner umfassend:
wenn ein Zentrum einer Läsionsregion in dem nasalen Endoskopiebild gesetzt ist, Extrahieren der Vielzahl von Pflasterbildern, die Teilregionen entsprechen, aus dem nasalen Endoskopiebild durch Beschneiden der Teilregionen basierend auf einem Zentrum der Läsionsregion.

5. Trainingsverfahren nach Anspruch 1, ferner umfassend:

Durchführen einer Bildverarbeitung an mindestens einem der extrahierten Pflasterbilder und dem nasalen Endoskopiebild, wobei die Bildverarbeitung eine Größenvergrößerung, eine Größenverkleinerung, eine Drehung oder eine Verschiebung mindestens umfasst, und
wobei das Trainieren des Läsionserkennungsmodells das Trainieren des Läsionserkennungsmodells basierend auf mindestens einem der extrahierten Pflasterbilder und dem nasalen Endoskopiebild, auf dem die Bildverarbeitung durchgeführt wird, umfasst.

6. Trainingsverfahren nach Anspruch 1, wobei das Läsionserkennungsmodell konfiguriert ist, um zu klassifizieren, ob das nasale Endoskopiebild ein Läsionsbild ist, das eine Läsionsregion umfasst, oder ein normales Bild, das keine Läsionsregion umfasst, und wobei das Bestimmungsmodell konfiguriert ist, um die Läsion als einen Nasenpolypen, ein umgekehrtes Papillom oder ein normales Gewebe zu klassifizieren.

7. Trainingsverfahren nach Anspruch 1, wobei das Trainieren des Läsionserkennungsmodells Folgendes umfasst:

   Zuweisen eines Gewichts zu einer Textur und einer Form einer Erfassungsregion der Nasenhöhle, die in mindestens einem der Bilder der Nasenendoskopie und der extrahierten Vielzahl von Pflasterbildern enthalten ist; und
   Durchführen eines Curriculum-Lernens des Läsionserkennungsmodells basierend auf der Textur und der Form der Erfassungsregion der Nasenhöhle, der das Gewicht zugewiesen ist.

8. Verfahren zur Bestimmung der Nasenhöhlenmasse, umfassend:

   Extrahieren einer Vielzahl von Pflasterbildern aus einem nasalen Endoskopiebild, umfassend eine Nasenhöhlenregion;
   Erkennen des Vorhandenseins einer Läsion, basierend auf mindestens einem der Bilder der Nasenendoskopie und der Vielzahl von Pflasterbildern unter Verwendung eines Läsionserkennungsmodells;
   wenn erkannt wird, dass die Läsion in dem nasalen Endoskopiebild vorhanden ist, Erzeugen eines kombinierten Bildes durch Kombinieren des nasalen Endoskopiebildes mit einem Computertomographiebild, CT, der Nasenhöhlenregion; und
   Durchführen, basierend auf dem erzeugten kombinierten Bild, der Bestimmung der Nasenhöhlenmasse für die Läsion unter Verwendung eines auf einem neuronalen Netzwerk basierenden Bestimmungsmodells.

9. Verfahren zur Bestimmung der Nasenhöhlenmasse nach Anspruch 8, wobei das nasale Endoskopiebild einem gesamten Bild entspricht, das die Region der Nasenhöhle umfasst, und
die Pflasterbilder Teilbildern des gesamten Bildes entsprechen.

10. Verfahren zur Bestimmung der Nasenhöhlenmasse nach Anspruch 8, wobei das Extrahieren der Vielzahl von Pflasterbildern das Extrahieren der Vielzahl von Pflasterbildern, die Teilregionen entsprechen, aus dem nasalen Endoskopiebild umfasst, indem die Teilregionen zufällig aus einer gesamten Region des nasalen Endoskopiebildes ausgeschnitten werden.

11. Verfahren zur Bestimmung der Nasenhöhlenmasse nach Anspruch 8, wobei das Läsionserkennungsmodell unter Verwendung des nasalen Endoskopiebildes und der Vielzahl von Pflasterbildern trainiert wird, und
nachdem sie trainiert wurde, konfiguriert, um zu klassifizieren, ob es sich bei dem nasalen Endoskopiebild um ein Läsionsbild handelt, das eine Läsionsregion umfasst, oder um ein normales Bild, das keine Läsionsregion umfasst.

12. Verfahren zur Bestimmung der Nasenhöhlenmasse nach Anspruch 8, wobei das Läsionserkennungsmodell konfiguriert ist, um einer Textur und einer Form einer Nasenhöhlenregion, die mindestens eines von dem Nasenendoskopiebild und der Vielzahl von Pflasterbildern umfasst, eine Gewichtung zuzuweisen und basierend auf der Textur und der Form der Nasenhöhlenregion, der die Gewichtung zugewiesen wird, trainiert wird.

13. Verfahren zur Bestimmung der Nasenhöhlenmasse nach Anspruch 8, wobei das auf einem neuronalen Netzwerk basierende Bestimmungsmodell basierend auf dem kombinierten Bild, das durch die Kombination des nasalen Endoskopiebildes mit einem CT-Bild der Nasenhöhlenregion erzeugt wurde, trainiert wird und nach dem Training konfiguriert ist, um die Läsion als einen Nasenpolypen, ein umgekehrtes Papillom oder ein normales Gewebe zu klassifizieren.

14. Trainingsgerät eines Modells zum Bestimmen einer Nasenhöhlenmasse, das Trainingsgerät umfassend:

   einen Bildvorverarbeitungsteil, der konfiguriert ist, um eine Vielzahl von Pflasterbildern aus einem nasalen Endoskopiebild zu extrahieren, das eine Nasenhöhlenregion umfasst;
   einen Trainingsteil für ein Läsionserkennungsmodell, der konfiguriert ist, um ein auf einem neuronalen Netzwerk basierendes Läsionserkennungsmodell unter Verwendung des nasalen Endoskopiebildes und der extrahierten Vielzahl von Pflasterbildern zu trainieren;

einen Detektor, der konfiguriert ist, um das Vorhandensein einer Läsion aus dem nasalen Endoskopiebild unter Verwendung des trainierten Läsionserkennungsmodells zu erkennen;

einen Generator für kombinierte Bilder, der konfiguriert ist, um, wenn erkannt wird, dass die Läsion in dem nasalen Endoskopiebild vorhanden ist, ein kombiniertes Bild zu erzeugen, indem das nasale Endoskopiebild mit einem Computertomographiebild der Nasenhöhlenregion kombiniert wird; und

einen Trainingsteil für ein Bestimmungsmodell, der konfiguriert ist, um basierend auf dem erzeugten kombinierten Bild ein auf einem neuronalen Netzwerk basierendes Bestimmungsmodell zu trainieren, um die Nasenhöhlemassen der Läsion zu bestimmen.

15. Gerät zur Bestimmung der Nasenhöhlenmasse, umfassend:

einen Bildvorverarbeitungsteil, der konfiguriert ist, um eine Vielzahl von Pflasterbildern aus einem nasalen Endoskopiebild zu extrahieren, das eine Nasenhöhlenregion umfasst; einen Detektor, der konfiguriert ist, um das Vorhandensein einer Läsion zu erkennen, basierend auf mindestens einem von dem nasalen Endoskopiebild und der Vielzahl von Pflasterbildern unter Verwendung eines Läsionserkennungsmodells;

einen Generator für kombinierte Bilder, der konfiguriert ist, um, wenn erkannt wird, dass die Läsion in dem nasalen Endoskopiebild vorhanden ist, ein kombiniertes Bild zu erzeugen, indem das nasale Endoskopiebild mit einem Computertomographiebild der Nasenhöhlenregion kombiniert wird; und

einen Bestimmungsteil für die Nasenhöhlenmasse, der konfiguriert ist, um die Bestimmung der Nasenhöhlenmasse auf der Läsion basierend auf dem erzeugten kombinierten Bild unter Verwendung eines auf einem neuronalen Netzwerk basierenden Bestimmungsmodells durchzuführen.

**Revendications**

1. Procédé d'entraînement d'un modèle permettant la détermination d'une masse de cavité nasale, le procédé d'entraînement comprenant :

l'extraction d'une pluralité d'images de patch à partir d'une image d'endoscopie nasale comprenant une région de cavité nasale ;

l'entraînement d'un modèle de détection de lésions basé sur un réseau neuronal à l'aide de l'image d'endoscopie nasale et de la pluralité d'images de patch ;

la détection d'une présence d'une lésion à partir de l'image d'endoscopie nasale à l'aide du modèle de détection de lésions entraîné ;

lorsqu'il est détecté que la lésion est présente sur l'image d'endoscopie nasale, la génération d'une image combinée en combinant l'image d'endoscopie nasale avec une image de tomodensitométrie de la région de cavité nasale ; et l'entraînement, sur la base de l'image combinée générée, d'un modèle de détermination basé sur un réseau neuronal permettant la détermination des masses de cavité nasale liées à la lésion.

2. Procédé d'entraînement selon la revendication 1, dans lequel

l'image d'endoscopie nasale correspond à une image entière comprenant la région de cavité nasale, et

les images de patch correspondent à des images partielles de l'image entière.

3. Procédé d'entraînement selon la revendication 1, dans lequel l'extraction de la pluralité d'images de patch comprend l'extraction, à partir de l'image d'endoscopie nasale, de la pluralité d'images de patch correspondant à des régions partielles en recadrant de manière aléatoire les régions partielles à partir d'une région entière de l'image d'endoscopie nasale.

4. Procédé d'entraînement selon la revendication 1, comprenant en outre :
lorsqu'un centre d'une région de lésion est défini sur l'image d'endoscopie nasale, l'extraction, à partir de l'image d'endoscopie nasale, d'une pluralité d'images de patch correspondant à des régions partielles en recadrant les régions partielles sur la base d'un centre de la région de lésion.

5. Procédé d'entraînement selon la revendication 1, comprenant en outre :

l'exécution d'un traitement d'image sur au moins l'une parmi les images de patch extraites et l'image d'endoscopie nasale, le traitement d'image comprenant au moins l'un des éléments suivants : l'agrandissement de taille, la réduction de taille, la rotation et le décalage, et

dans lequel l'entraînement du modèle de détection de lésions comprend l'entraînement du modèle de détection de lésions sur la base d'au moins l'une parmi les images de patch extraites et l'image d'endoscopie nasale sur laquelle le traitement d'image est exécuté.

6. Procédé d'entraînement selon la revendication 1, dans lequel le modèle de détection de lésions est configuré pour classer si l'image d'endoscopie nasale est une image de lésion comprenant une région de lésion ou une image normale ne comprenant pas de région de lésion, et

   dans lequel le modèle de détermination est configuré pour classer la lésion comme l'un parmi un polype nasal, un papillome inversé ou un tissu normal.

7. Procédé d'entraînement selon la revendication 1, dans lequel l'entraînement du modèle de détection de lésions comprend :

   l'attribution d'un poids à une texture et à une forme d'une région de cavité nasale comprise dans au moins l'une parmi l'image d'endoscopie nasale et la pluralité d'images de patch extraites ; et
   l'exécution, sur la base de la texture et de la forme de la région de cavité nasale à laquelle le poids est attribué, d'un apprentissage structuré sur le modèle de détection de lésions.

8. Procédé de détermination de masse de cavité nasale comprenant :

   l'extraction d'une pluralité d'images de patch à partir d'une image d'endoscopie nasale comprenant une région de cavité nasale ;
   la détection d'une présence d'une lésion sur la base d'au moins l'une parmi l'image d'endoscopie nasale et la pluralité d'images de patch à l'aide d'un modèle de détection de lésions ;
   lorsqu'il est détecté que la lésion est présente dans l'image d'endoscopie nasale, la génération d'une image combinée en combinant l'image d'endoscopie nasale avec une image de tomodensitométrie, TDM, de la région de cavité nasale ; et
   l'exécution, sur la base de l'image combinée générée, d'une détermination de masse de cavité nasale pour la lésion à l'aide d'un modèle de détermination basé sur un réseau neuronal.

9. Procédé de détermination de masse de cavité nasale selon la revendication 8, dans lequel l'image d'endoscopie nasale correspond à une image entière comprenant la région de cavité nasale, et
   les images de patch correspondent à des images partielles de l'image entière.

10. Procédé de détermination de masse de cavité nasale selon la revendication 8, dans lequel l'extraction de la pluralité d'images de patch comprend l'extraction, à partir de l'image d'endoscopie nasale, de la pluralité d'images de patch correspondant à des régions partielles en recadrant de manière aléatoire les régions partielles à partir d'une région entière de l'image d'endoscopie nasale.

11. Procédé de détermination de masse de cavité nasale selon la revendication 8, dans lequel le modèle de détection de lésions est entraîné à l'aide de l'image d'endoscopie nasale et de la pluralité d'images de patch, et
   une fois entraîné, est configuré pour classer si l'image d'endoscopie nasale est une image de lésion comprenant une région de lésion ou une image normale ne comprenant pas de région de lésion.

12. Procédé de détermination de masse de cavité nasale selon la revendication 8, dans lequel le modèle de détection de lésions est configuré pour attribuer un poids à une texture et à une forme d'une région de cavité nasale comprise dans au moins l'une parmi l'image d'endoscopie nasale et la pluralité d'images de patch et est entraîné sur la base de la texture et de la forme de la région de cavité nasale à laquelle le poids est attribué.

13. Procédé de détermination de masse de cavité nasale selon la revendication 8, dans lequel le modèle de détermination basé sur un réseau neuronal est entraîné à partir de l'image combinée générée en combinant l'image d'endoscopie nasale avec une image TDM de la région de cavité nasale, et une fois entraîné, est configuré pour classer la lésion comme l'un parmi un polype nasal, un papillome inversé ou un tissu normal.

14. Appareil d'entraînement d'un modèle permettant la détermination d'une masse de cavité nasale, l'appareil d'entraînement comprenant :

    une partie de prétraitement d'image configurée pour extraire une pluralité d'images de patch à partir d'une image d'endoscopie nasale comprenant une région de cavité nasale ;
    une partie d'entraînement de modèle de détection de lésions configurée pour entraîner un modèle de détection de lésions basé sur un réseau neuronal à l'aide de l'image d'endoscopie nasale et de la pluralité d'images de patch extraites ;
    un détecteur configuré pour détecter une présence d'une lésion à partir de l'image d'endoscopie nasale à l'aide du modèle de détection de lésions entraîné ;
    un générateur d'images combinées configuré pour, lorsqu'il est détecté que la lésion est présente sur l'image d'endoscopie nasale, générer une image combinée en combinant l'image d'endoscopie nasale avec une image de tomodensitométrie de la région de cavité nasale ; et une partie d'entraînement de modèle de détermination configurée pour entraîner, sur la base de l'image combinée générée, un modèle de détermination basé sur un réseau neuronal permettant la détermination des masses de cavité nasale de la lésion.

15. Appareil de détermination de masse de cavité nasale comprenant :

une partie de prétraitement d'image configurée pour extraire une pluralité d'images de patch à partir d'une image d'endoscopie nasale comprenant une région de cavité nasale ; un détecteur configuré pour détecter une présence d'une lésion sur la base d'au moins l'une parmi l'image d'endoscopie nasale et la pluralité d'images de patch à l'aide d'un modèle de détection de lésions ;
un générateur d'images combinées configuré pour, lorsqu'il est détecté que la lésion est présente sur l'image d'endoscopie nasale, générer une image combinée en combinant l'image d'endoscopie nasale avec une image de tomodensitométrie de la région de cavité nasale ; et une partie de détermination de masse de cavité nasale configurée pour exécuter une détermination de masse de cavité nasale sur la lésion sur la base de l'image combinée générée à l'aide d'un modèle de détermination basé sur un réseau neuronal.

CT image
130

120

Nasal
endoscopy image
110

Lesion detection
model
140

Determination
model
150

Determination
result
160

FIG. 1

Start

210
Extract plurality of patch images from nasal endoscopy image
including nasal cavity region

220
Train neural network-based lesion detection model using nasal endoscopy image
and extracted plurality of patch images

230
Detect presence of lesion from nasal endoscopy image using trained
lesion detection model

240
When it is detected that lesion is present in nasal endoscopy image,
generate combined image by combining nasal endoscopy image with
CT image of nasal cavity region

250
Train, based on generated combined image, neural network-based
determination model for determining nasal cavity mass for lesion

End

FIG. 2

```
                          ┌──────────────┐
                          │    Start     │
                          └──────┬───────┘
                                 │                      ╱ 310
         ┌───────────────────────▼───────────────────────────┐
         │  Extract plurality of patch images from nasal      │
         │  endoscopy image including nasal cavity region     │
         └───────────────────────┬───────────────────────────┘
                                 │                      ╱ 320
         ┌───────────────────────▼───────────────────────────┐
         │  Detect presence of lesion based on at least one   │
         │  of nasal endoscopy image and plurality of patch   │
         │  images using lesion detection model               │
         └───────────────────────┬───────────────────────────┘
                                 │                      ╱ 330
         ┌───────────────────────▼───────────────────────────┐
         │  When it is detected that lesion is present in     │
         │  nasal endoscopy image, generate combined image    │
         │  by combining nasal endoscopy image with           │
         │  CT image of nasal cavity region                   │
         └───────────────────────┬───────────────────────────┘
                                 │                      ╱ 340
         ┌───────────────────────▼───────────────────────────┐
         │  Perform nasal cavity mass determination on lesion │
         │  based on generated combined image using neural    │
         │  network-based determination model                 │
         └───────────────────────┬───────────────────────────┘
                                 │
                          ┌──────▼───────┐
                          │     End      │
                          └──────────────┘
```

## FIG. 3

FIG. 4

**FIG. 5**

EP 4 579 685 B1

600

| | | |
|---|---|---|
| **Image preprocessing part** 610 | **Lesion detection model training part** 620 | **Detector** 630 |
| **Combined image generator** 640 | **Determination model training part** 650 | |

## FIG. 6

700

| | |
|---|---|
| **Image preprocessing part** 710 | **Detector** 720 |
| **Combined image generator** 730 | **Nasal cavity mass determiner** 740 |

## FIG. 7

21